# EUROPEAN PATENT SPECIFICATION

(11) **EP 3 223 601 B1**
(45) Date of publication and mention of the grant of the patent: **30.12.2020**
(21) Application number: 15862304.1
(22) Date of filing: 26.11.2015
(51) Int. Cl.: A01H 1/02

(54) **POLLEN APPLICATOR**
POLLENAPPLIKATOR
APPLICATEUR DE POLLEN

(30) Priority: 28.11.2014 NZ 70244914
(43) Date of publication of application: 04.10.2017
(73) Proprietor: Ovink, Willem Hendrik Klein, Whakatane 3192 (NZ); Horwood, David, Opotiki 3198 (NZ)
(72) Inventor: HORWOOD, David, Opotiki, 3198 (NZ)
(74) Representative: Gallego Jiménez, José Fernando
(86) International application number: PCT/NZ2015/050199
(87) International publication number: WO 2016/085355

(56) References cited:
- FR-A1- 2 404 390
- FR-A1- 2 866 784
- FR-A1- 2 979 798
- KR-B1- 101 441 357
- NZ-A- 214 514
- US-A- 1 306 209
- US-A- 2 324 801
- US-A- 4 087 937
- US-A- 4 922 651
- US-A1- 2006 131 444
- M. J. KING ET AL: "Collection and use of dry pollen for pollination of kiwifruit", NEW ZEALAND JOURNAL OF CROP AND HORTICULTURAL SCIENCE., vol. 19, no. 4, 1 October 1991 (1991-10-01), pages 385-389, XP055459227, NZ ISSN: 0114-0671, DOI: 10.1080/01140671.1991.10422881
- Michael J W Hii: "Kiwifruit Flower Pollination - Wind Pollination Efficiencies and Sprayer Jet Applications Chemical and Process Engineering", Thesis, 1 January 2004 (2004-01-01), pages 1-323, XP055258644, Christchurch, New Zealand Retrieved from the Internet: URL:www.ir.canterbury.ac.nz/bitstream/1009 2/6535/1/hii_thesis.pdf [retrieved on 2016-03-16]

## Description

### FIELD OF INVENTION

One aspect of the invention relates to applying pollen in a horticultural setting.

### BACKGROUND

Pollination is an essential part of growing fruit. For fruit to grow its flowers must be pollinated. If pollination does not occur then normally no fruit will grow. If poor pollination occurs, then the resulting fruit may be of poor quality and may be rejected for sale by fruit wholesalers. Bees play an important part in pollenating flowers of many types. However, there is a global decline in bee numbers and they cannot always be relied on to provide good pollination. Bees also prefer certain flowers over others; for example they are often not particularly attracted to kiwifruit flowers.

Good pollination in kiwifruit is critical for good fruit size and shape. Kiwifruit have male flowers and female flowers. To achieve good pollination in Hayward kiwifruit, female flowers need to be within 5m of male flowers if bees are the only source of pollination.

Many orchardists supplement bees with artificial pollination. Male flowers are picked and milled and the pollen then reapplied to the female flowers by either dry or wet application techniques. Typically, one pass with a dry pollen blower pollinates 33% of the fruit ovules. The other 66% of ovules are pollinated by bees moving the applied pollen between flowers or within flowers.

It has been found that rates of over 250g of pollen applied per hectare do not appear to improve the pollination outcome. More than 90% of pollen dispersed by current dry pollen applicators get nowhere near the female flowers and is wasted.

Pollen is very expensive (approximately $3000 per kg) and in some years is in short supply. Excellent artificial pollination can currently be achieved manually if every flower is dosed with a hand held applicator, either touching, or within 10cm of every flower (hand held applicators include pollen rollers, puffers or sprayers). These methods are labour intensive and costly. In the case of wet application of pollen, the costs can exceed $6000 per hectare.

The arrival of PSA (Pseudomonas syringae actinidiae) is causing bud rot in kiwifruit flower, particularly males so sometimes there is not enough male pollen within an orchard, or at the ideal distribution.

More and more kiwifruit orchards are now being covered for a variety of reasons and bees are often reluctant to work under these canopies. Bees can also fatally damage their wings on these canopies and some bee keepers will not put their hives in covered orchards.

PSA (Pseudomonas syringae actinidiae) is a bacteria that affects kiwifruit and can lead to the death of kiwifruit vines. PSA only affects kiwifruit. It is believed to be spread by wind, rain and plant materials. It may also be spread by footwear, vehicles and orchard equipment.

It is an object of a preferred form of the invention to go at least some way towards facilitating pollination of plant life.

King and Ferguson (1991) disclose a boomsprayer for pollinating kiwi flowers.

Hii, M.J.W. (Thesis, University of Canterbury, 2004) discloses the use of single or horizontal air jets for kiwi flower pollination.

### SUMMARY OF THE INVENTION

In one embodiment of the invention there is a pollen applicator for blowing pollen towards plant life when in use, the applicator comprising:
- a blower adapted to blow pollen into the air in the vicinity of plants while the applicator moves past a series of the plants; and
- a vacuum collector adapted to capture some of the blown pollen and recycle it to be blown again by the blower.

In some embodiments more than one blower is provided.

Preferably the pollen applicator further comprises a pollen source adapted to provide a measured amount of pollen to a blower.

Preferably pollen captured by the vacuum collector is recycled to all the blowers.

Preferably the pollen applicator further comprises a fan to provide air to the blower(s).

Preferably the pollen applicator further comprises a trailer adapted to be towed.

In some embodiments the pollen applicator further comprises a motive unit to move the applicator.

Preferably pollen lingering near the applicator after being blown by one of the blowers is subsequently blown by another one of the blowers.

In another aspect the invention comprises a method of blowing pollen towards plant life comprising the steps of providing pollen to a pollen applicator for blowing pollen towards plant life when in use, the applicator comprising: a blower adapted to blow pollen into the air in the vicinity of plants while the applicator moves past a series of the plants; and a vacuum collector adapted to capture some of the blown pollen and recycle it to be blown again by the blower; activating the blower to blow a measured amount of pollen, activating the vacuum collector, and towing the pollen applicator past a series of plants to be pollinated.

In another aspect the invention comprises a pollen applicator for blowing pollen towards plant life when in use, the applicator having a series of blowers adapted to blow pollen into the air in the vicinity of plants while moving past a series of plants, the applicator formed such that any pollen lingering near the applicator after being blown by one of the blowers is subsequently blown by another one of the blowers.

Optionally the blowers comprise outlets associated with a common fan.

### IMAGES

Some preferred forms of the invention will now be described by way of example and with reference to the accompanying images, of which:
**Figure 1** is an isometric view of a pollen applicator; and
**Figure 2** is an alternative isometric view of the same applicator.

### DETAILED DESCRIPTION

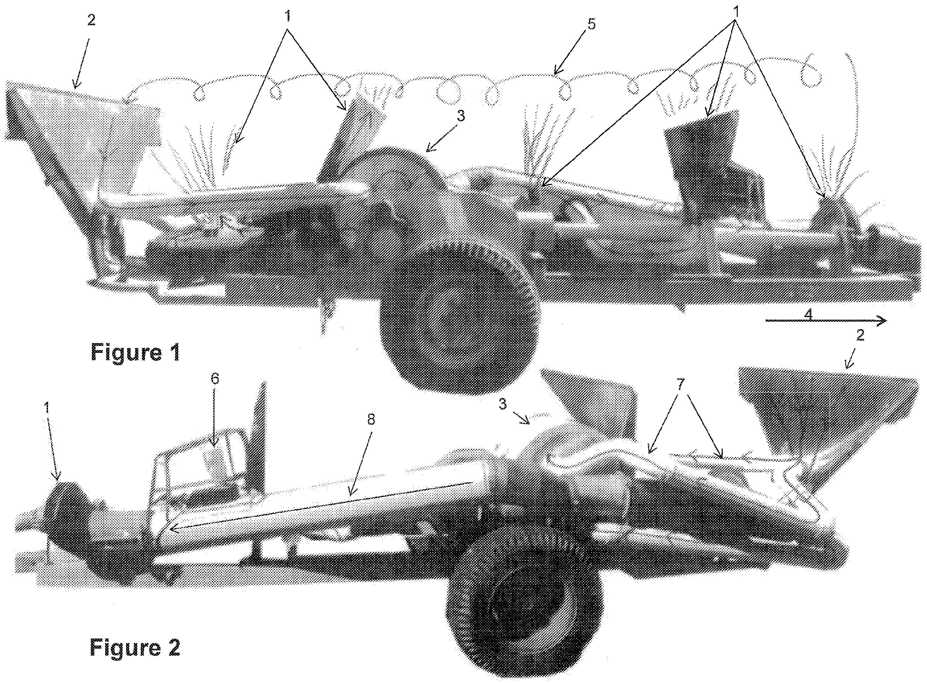

Figures 1 and 2 show a pollen applicator including blowers 1, a vacuum collector 2, a pollen source 6 (shown in figure 2) and a fan 3, mounted on a trailer (the trailer may be part of the applicator).

The trailer can be coupled to a vehicle to allow the applicator to be moved past a series of plants, for example kiwifruit vines. In this regard the applicator it may be towed by a quad bike, tractor or some other vehicle. In alternative embodiments the applicator may have its own motive unit so it can be driven, ie without needing to be towed.

In some embodiments a single blower is provided. The blower 1 blows pollen provided by the pollen source 6 into the air. As the applicator moves past the plants the pollen is blown into the air to pollinate flowers belonging to the plants.

In some embodiments the pollen applicator is provided with a vacuum collector 2. The vacuum collector 2 is arranged to suck in air and unused (eg lingering) pollen as the applicator moves past a series of plants in a forward direction 4 (indicated in figure 1). The collector 2 therefore provides air and pollen to the fan 3 via conduits 7. The fan 3 creates suction for the vacuum collector 2. The air from the fan 3 is then provided back to at least one of the blowers 1 along the conduit as shown at 8 to recycle any unused pollen that has been collected by vacuum collector 2.

As indicated above, pollen is provided by the pollen source 6. The pollen source 6 dispenses a measured amount of pollen to a first of the blowers 1 as indicated at 8 (see figure 2). That blower then blows the pollen into the air. As the applicator moves past a series of plants in the forward direction 4 pollen is blown into the air and drifts towards the plants.

In some embodiments more than one blower 1 is provided. In some embodiments pollen from pollen source 6 is provided to one of the blowers 1 closest to the front of the applicator (as determined by the direction in which the pollen applicator will be moved). The pollen is blown from this blower into the air. Blowers behind the first blower then blow air and any pollen that is lingering or falling towards the ground upwards as the pollen applicator moves in direction 4. Some pollen may follow path 5 shown in figure 1, being blown by the first blower and then subsequent blowers. In this embodiment the blowers 1 all receive air flow from the same fan 3, but in alternative embodiments there may be more than one fan. The subsequent blowers may be arranged to blow pollen in different directions (as shown in figure 1) to provide a greater coverage of pollen to the plants. Unused pollen may be collected by vacuum collector 2 and recycled to all the blowers by way of fan 3.

When the pollen applicator includes a vacuum collector 2, the vacuum collector collects any pollen it can. This pollen is provided to fan 3 by conduits 7 and dispensed to all the blowers 1 by a series of conduits. The conduits may be formed by any suitable pipes, tubes or the like.

In use the pollen applicator is moved past a series of plants to be pollinated in direction 4. The pollen source 6 provides pollen to the first blower 1. Pollen blown by first blower 1 may pollinate flowers on the plants. If the pollen doesn't pollinate the flower it may start to fall to the ground. In some embodiments further blowers may blow the falling pollen towards plants. In some embodiments some of the falling pollen may be collected by vacuum collector 2 and recycled to the blower(s) by fan 3.

In a kiwifruit orchard the blowers are directed at the female kiwifruit flowers. As the machine passes by, each female kiwifruit flower will have pollen blown at it multiple times (in the applicator shown in the images, 5 times). If each of the blowers pollinates 33% of the kiwifruit ovules, then one pass of the pollen applicator should achieve perfect pollination.

When provided, the vacuum collector of the applicator at the rear of the applicator (when viewed in the direction of movement) collects and recycles any pollen floating within the width of the machine and up to 25cms vertically above and below the vacuum inlet (give half a meter height coverage in total). The pollen collected by the vacuum is then recirculated and redistributed through the multiple blowers. Using a vacuum collector has the potential to allow a reduced amount of pollen to be dispensed at the of the machine and still achieve perfect pollination at rates of less than 250g of pollen per hectare.

In the claims which follow and in the preceding description of the invention, except where the context requires otherwise due to express language or necessary implication, the word "comprise" or variation such as "comprises" or "comprising" is used in an inclusive sense, i.e. to specify the presence of the stated features but not to preclude the presence or addition of further features in various embodiments of the invention.

While some preferred forms of the invention have been described by way of example it should be appreciated that modifications can occur without departing from the scope of the invention as defined in the accompanying claims.

## Claims

1. A pollen applicator blowing pollen towards plant life, the applicator having a pollen source adapted to provide a measured amount of pollen, a series of blowers, and a vacuum collector, arranged such that:
• the pollen source provides pollen to one of the blowers;
• the blowers blow the pollen into the air in the vicinity of plants while the applicator moves past a series of the plants;
• the vacuum collector captures some of the blown pollen and recycles it to be blown again;
• pollen lingering near the applicator after being blown by one of the blowers is subsequently blown by another one of the blowers; and
• the pollen applicator:
∘ is mounted on a trailer; or
∘ incorporates a trailer; or
∘ has a motive unit so it can be driven without needing to be towed.

2. A pollen applicator as claimed in claim 1, comprising a fan that provides air to the blower(s).

3. A pollen applicator as claimed in claim 1 or claim 2, comprising a trailer that is towed.

4. A pollen applicator as claimed in claim 1 or claim 2, comprising a motive unit that moves the applicator.

5. A pollen applicator as claimed in any one of the previous claims, wherein the blowers comprise outlets associated with a common fan.

## Patentansprüche

1. Pollenapplikator, der Pollen auf Pflanzenleben bläst, wobei der Applikator eine Pollenquelle, die ausgelegt ist, eine gemessene Pollenmenge bereitzustellen, eine Reihe von Gebläsen und einen Vakuumsammler aufweist, der derart angeordnet ist, dass:
• die Pollenquelle einem der Gebläse Pollen bereitstellt;
• die Gebläse den Pollen in der Nähe von Pflanzen in die Luft blasen, während sich der Applikator an einer Reihe von Pflanzen vorbei bewegt;
• der Vakuumsammler einen Teil des geblasenen Pollens auffängt und ihn zurückführt, damit er erneut geblasen wird;
• Pollen, die in der Nähe des Applikators verbleiben, nachdem sie von einem der Gebläse geblasen wurden, anschließend von einem anderen Gebläse geblasen werden; und
• der Pollenapplikator:
∘ auf einem Anhänger montiert ist; oder
∘ einen Anhänger integriert; oder
∘ eine Antriebseinheit aufweist, so dass er gefahren werden kann, ohne dass er gezogen werden muss.

2. Pollenapplikator nach Anspruch 1, umfassend einen Lüfter, der dem(den) Gebläse(n) Luft bereitstellt.

3. Pollenapplikator nach Anspruch 1 oder Anspruch 2, umfassend einen Anhänger, der gezogen wird.

4. Pollenapplikator nach Anspruch 1 oder Anspruch 2, umfassend eine Antriebseinheit, die den Applikator bewegt.

5. Pollenapplikator nach einem der vorhergehenden Ansprüche, wobei die Gebläse Auslässe umfassen, die einem gemeinsamen Lüfter zugeordnet sind.

## Revendications

1. Applicateur de pollen soufflant du pollen vers la flore, l'applicateur ayant une source de pollen adaptée pour fournir une quantité mesurée de pollen, un ensemble de souffleurs et un collecteur sous vide, disposés de telle sorte que :
• la source de pollen fournit du pollen à l'un des souffleurs ;
• les souffleurs soufflent le pollen dans l'air à proximité des plantes pendant que l'applicateur passe devant un ensemble des plantes ;
• le collecteur sous vide capte une partie du pollen soufflé et la recycle pour être à nouveau soufflée ;
• le pollen persistant près de l'applicateur après avoir été soufflé par l'un des souffleurs est ensuite soufflé par un autre des souffleurs ; et
• l'applicateur de pollen :
∘ est monté sur une remorque ; ou
∘ incorpore une remorque ; ou
∘ a une unité motrice afin qu'il puisse être conduit sans avoir besoin d'être tracté.

2. Applicateur de pollen selon la revendication 1, comprenant un ventilateur qui fournit de l'air au(x) souffleur(s).

3. Applicateur de pollen selon la revendication 1 ou la revendication 2, comprenant une remorque qui est tractée.

4. Applicateur de pollen selon la revendication 1 ou la revendication 2, comprenant une unité motrice qui déplace l'applicateur.

5. Applicateur de pollen selon l'une quelconque des revendications précédentes, dans lequel les souffleurs comprennent des sorties associées à un ventilateur commun.
